(19) Europäisches Patentamt
European Patent Office
Office européen des brevets

(11) **EP 2 380 532 B1**

(12) **EUROPEAN PATENT SPECIFICATION**

(45) Date of publication and mention
of the grant of the patent:
**03.12.2014 Bulletin 2014/49**

(51) Int Cl.:
***A61F 5/451*** *(2006.01)*

(21) Application number: **11168588.9**

(22) Date of filing: **17.09.2009**

(54) **Urine receiver and wearing article**

Urinfänger und Kleidungsstück damit

Récepteur d'urine et article vestimentaire

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **19.09.2008 JP 2008241776**
**19.09.2008 JP 2008241778**
**19.09.2008 JP 2008241779**

(43) Date of publication of application:
**26.10.2011 Bulletin 2011/43**

(62) Document number(s) of the earlier application(s) in accordance with Art. 76 EPC:
**09814633.5 / 2 335 659**

(73) Proprietor: **Unicharm Corporation**
**Ehime 799-0111 (JP)**

(72) Inventors:
• **Wada, Ichiro**
**Kanonji-shi, Kagawa 769-1602 (JP)**
• **Suzuki, Miou**
**Kanonji-shi, Kagawa 769-1602 (JP)**
• **Yamaki, Rumi**
**Kanonji-shi, Kagawa 769-1602 (JP)**
• **Murakami, Hiroko**
**Kanonji-shi, Kagawa 769-1602 (JP)**

(74) Representative: **Knights, Rupert**
**Saunders & Dolleymore LLP**
**9 Rickmansworth Road**
**Watford, Hertfordshire WD18 0JU (GB)**

(56) References cited:
**EP-A1- 1 457 178      EP-A1- 1 504 737**
**EP-A1- 1 520 566      WO-A2-2006/062828**
**US-A1- 2007 038 194**

**Description**

TECHNICAL FIELD

The present invention relates to wearing articles and more particularly to wearing articles such as pants, disposable diapers or incontinent briefs provided with a suction unit for suction of moisture such as urine.

RELATED ART

Conventionally, wearing articles such as pants provided on their side facing the wearer's skin with a moisture-suction system are known, for example, fromJP 08-510924 A. In JP 08-510924 A, a catheter system functioning to such and to evacuate urine outward from pants is described. In the case of this catheter system, the pants are provided on the side facing the wearer's body with an interface device comprising a plastic shell serving to receive urine, a liquid-impervious layer lying on the side of the shell facing the wearer's body and a hydrophobic cover stock material lying on the side of the liquid-impervious layer facing the wearer's body. A drain tube is connected to the shell so that urine staying in the pants may be collected into the shell under a suction effect and the urine collected into the shell may be evacuated from the pants through the drain tube.

DISCLOSURE OF THE INVENTION

PROBLEM TO BE SOLVED BY THE INVENTION

In the urine receiver disclosed in JP 2006-26108 A, a pair of electrodes is covered with the liquid-pervious sheet so that, upon occurrence of urination by the wearer of the urine receiver, urine may spread over this liquid-pervious sheet and/or the other liquid-pervious sheets and the paired electrodes may be electrically connected to each other via the liquid-pervious sheet (s) wetted with urine. However, it is not necessarily assured depending on the wearer's posture that the discharged urine smoothly spreads over the liquid-pervious sheet(s) and, in consequence, there is a possibility that the vacuum pump could not be actuated even when urination occurs.

The shell described in JP 08-510924 A is made of a plastic material and therefore liquid-impervious. In addition, the liquid-impervious layer is formed on the side of the shell facing the wearer's body. Certainly, discharged urine is sucked away from the shell but a small quantity of urine is inevitably left in the shell without being sucked away. Vaporization of this small quantity of urine raises a temperature within the pants and makes the inside of the pants stuffy. Sweat of the wearer also may raise a temperature within the pants and cause stuffiness. Stuffiness generated within the pants may induce skin troubles such as eczema and create a feeling of discomfort against the wearer.

A further prior art urine receiver is known from US 2007/0038194, which is regarded as the closest prior art.

It is an object of the present invention to assure the inside of the wearing article to be prevented from becoming stuffy and from causing skin troubles even when humidity within the wearing article rises.

According to the present invention, there is provided a wearing article as recited by Claim 1.

According to one embodiment of the present invention, the receiver additionally includes a moisture detecting structure comprising a moisture detecting sensor, a spacer lying on the side of the moisture detecting sensor facing the wearer's body, a dispersant sheet lying on the side of the moisture detecting sensor facing the wearer' s garment and an air permeable-resistant sheet lying on the side of the dispersant sheet facing the wearer's garment.

According to another embodiment of the present invention, the moisture-absorbent structure has a length in the transverse direction dimensioned to be longer than that of the moisture detecting structure to extend outward in the transverse direction beyond the side edges of the moisture detecting structure.

According to still another embodiment of the present invention, the moisture detecting structure extends in the longitudinal direction from the front waist region to the rear waist region.

According to one embodiment of the present invention, the receptacle is liquid-impervious and flexible and may position in the rear waist region in use of the receptacle.

EFFECT OF THE INVENTION

According to the present invention, the receiver includes the moisture-absorbent structure serving to absorb moisture vapor generated within the wearing article. With such an arrangement, even if moisture vapor within the wearing article increases, such moisture vapor can be absorbed by the moisture-absorbent structure and thereby it is possible to restrict a rise of the temperature within the wearing article from rising. It is possible thereby to prevent the interior of the wearing article from becoming stuffy as well as to reduce a feeling of discomfort against the wearer.

BRIEF DESCRIPTION OF THE DRAWINGS

Fig. 1 is an overall diagram schematically illustrating the system according to the present invention.
Fig. 2 is a plan view of the urine receiver as viewed from its side facing the wearer's body.
Fig. 3 is a sectional view taken along line III-III Fig. 2.
Fig. 4 is a sectional view taken along line IV-IV in Fig.2
Fig. 5 is a plan view of the urine receiver as viewed from its side facing the wearer's garment.
Fig. 6 is a perspective view of a urine receptacle.
Fig. 7 is a sectional view taken along line VII-VII in Fig. 6.

DETAILED DESCRIPTION OF THE PREFERRED EMBODIMENTS

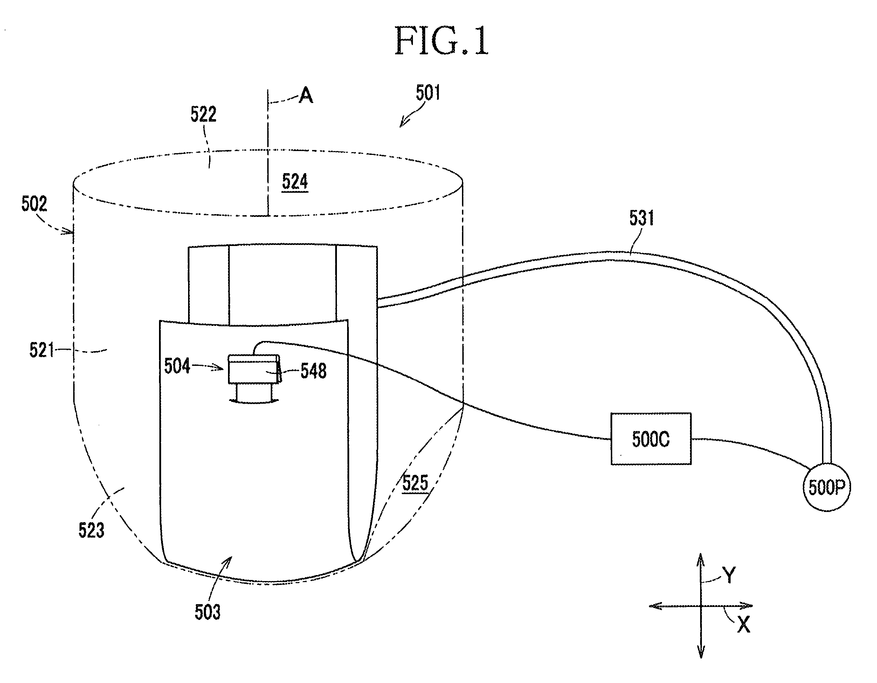

Fig. 1 is an overall diagram schematically illustrating a wearing article 501 according to the present invention. According to the present invention, a pants-type chassis 502 is formed therein with a urine receiver 503 to which a suction pump 500P as a suction unit via suction tube 531. The urine receiver 503 is electrically connected to the suction pump 500P which is adapted to be actuated by a control unit 500C when a moisture detecting structure 504 incorporated in the urine receiver 503 detects occurrence of urination. Upon actuation of the suction pump 500P, the urine collected by the urine receiver 503 is sucked and then evacuated out from the chassis 502. In Fig. 1, the chassis 502 is indicated by imaginary lines.

The chassis 502 comprises a front waist region 521, a rear waist region 522 and a crotch region 523 extending between these front and rear waist regions 521, 522 wherein these three regions are continuous in the longitudinal direction Y. The chassis 502 is formed of an elastic mesh sheet so that the chassis 502 may be elasticized in the longitudinal direction Y and in the transverse direction X which is orthogonal to the longitudinal direction Y. Particularly along peripheries of a waist-opening 524 and leg-openings 525, the elasticity is locally heightened to improve a fit of the chassis 502 to the wearer's waist and thighs, respectively.
On the side of the chassis 502 facing the wearer's body, the urine receiver 503 extends in the longitudinal direction Y from the front waist region 521 to the rear waist region 522 along a longitudinal center line A-A bisecting a dimension of the chassis 502 in the transverse direction X. The urine receiver 503 is kept in close contact with the wearer's body under the effect of elasticity of the chassis 502 so that only the urine receiver 503 may be exchanged as the need arises.

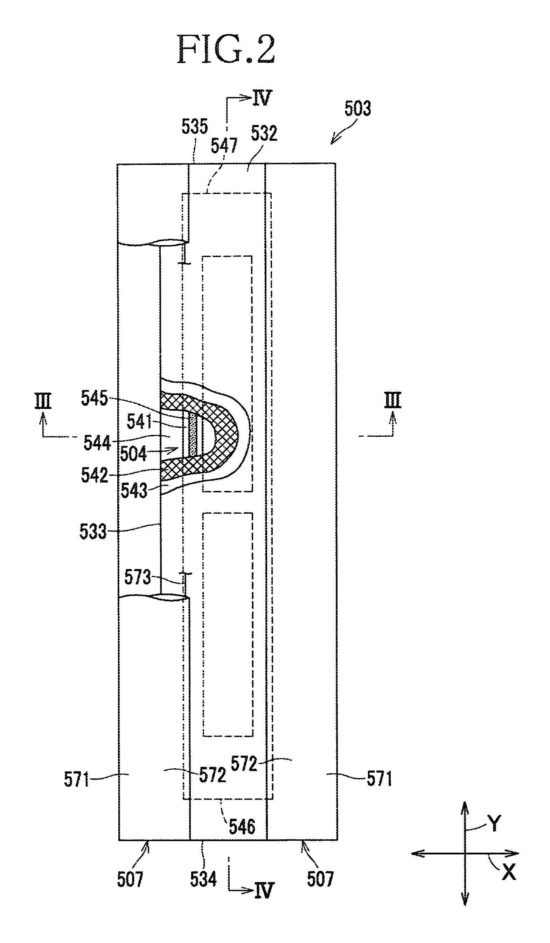

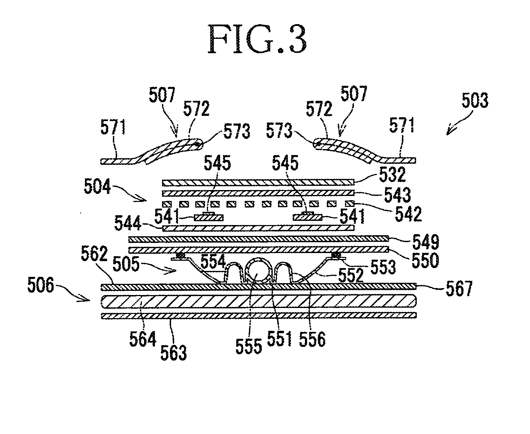

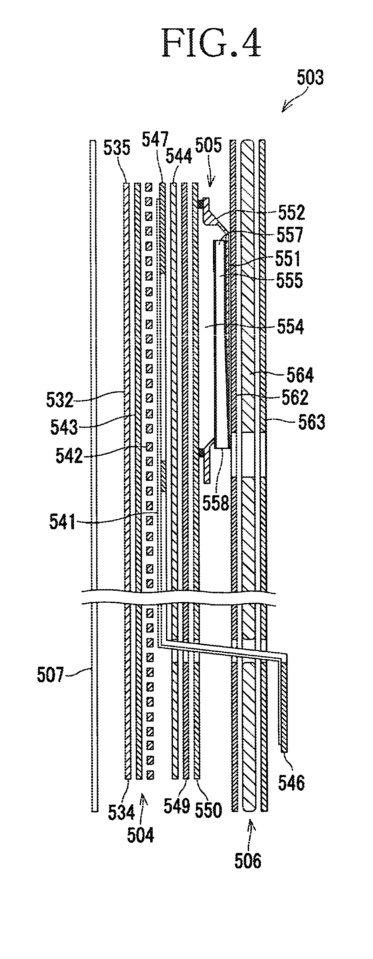

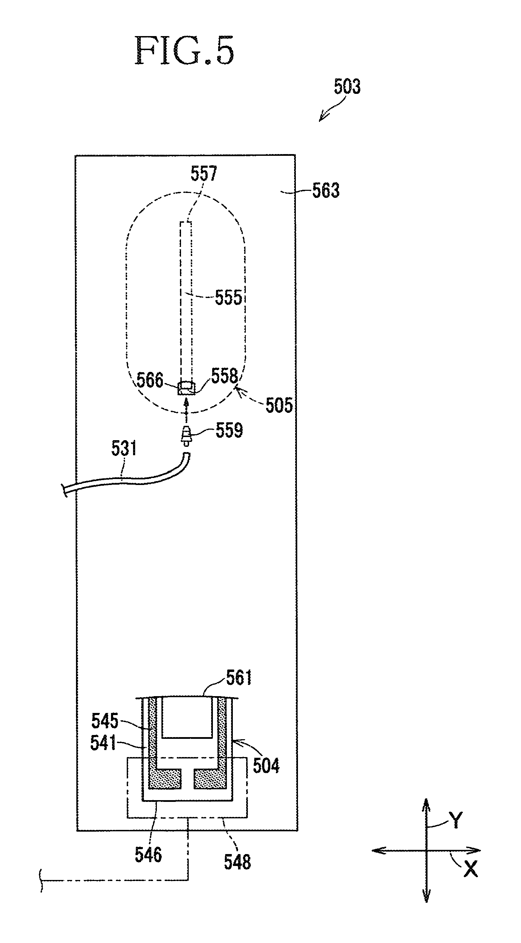

Fig. 2 is a plan view of the urine receiver 503 as viewed from its side facing the wearer's body. It should be appreciated that the urine receiver 503 is shown as partially cutaway and the chassis 502 is not shown at all for convenience of explanation. Fig. 3 is a sectional view taken along line III-III in Fig. 2 and Fig. 4 is a sectional view taken along IV-IV line in Fig.2 . While the respective sheets are illustrated to be spaced one from another for convenience of explanation in Figs. 3 and 4, these sheets are normally joined together. Fig. 5 is a plan view of the urine receiver of Fig. 2 as viewed from the side facing the wearer's garment.

The urine receiver 503 comprises a topsheet 532 adapted to come in contact with the wearer's body, a moisture detecting structure 504 serving to detect urination, a tray-shaped urine receptacle 505 located on the side of the moisture detecting structure 504 facing the wearer's body to receive discharged urine and a moisture absorbent structure 506 lying on the side of the urine receptacle 505 facing the wearer's garment to absorb moisture vapor in the chassis 502. The topsheet 532 may be formed, for example, of a liquid-pervious fibrous nonwoven fabric. The topsheet 532 has a generally rectangular shape extending in the longitudinal direction Y and includes opposite side edges 533, 533 extending in the longitudinal direction Y and front and rear ends 534, 535 extending in the transverse direction X.

The moisture detecting structure 504 extends the front waist region 521 to the rear waist region 522 of the chassis 502. The moisture detecting structure 504 comprises a pair of moisture detecting sensors 541, a spacer 542 lying on the side of the moisture detecting sensors 541 facing the wearer's body, a filter 543 lying on the side of the spacer 542 facing the wearer's body and a cushion sheet 544 lying on the side of the moisture detecting sensors 541 facing the wearer's garment. Specifically, the moisture detecting sensors 541 is sandwiched between the spacer 542 and the cushion sheet 544 in the thickness direction and thereby protected from any external shock.

The moisture detecting sensors 541 are formed of a pair of electrodes 545, 545 spaced from each other in the transverse direction X and these electrodes 545, 545 are obtained, for example, by printing a thermoplastic synthetic film such as a polyethylene film with a conductive coating material. These electrodes 545, 545 extend in parallel to each other in the longitudinal direction Y. The moisture detecting sensors 541 respectively have front and rear ends 546, 547 extending in the transverse direction X wherein the sensor's front end 546 extends through a slit 561 formed in the moisture absorbent structure 506 to the side thereof facing the wearer's garment. The slit 561 is formed on the side of the front waist region 521. The sensor's front end 54 6 extending out through the slit 561 is provided with a clip 548 via which the signal from the moisture detecting sensors 541 is transmitted to the control unit 500C.

More specifically, the moisture detecting sensors 541 are turned on in response to electrical connection of the paired electrodes 545, 545 effectuated by the present of urine therebetween and the control unit 500C detects occurrence of urination on the basis of this turned-on state.

As a material for the spacer 542, a mesh sheet having high air- and liquid-permeability and serves to keep the filter 543 and the moisture detecting sensors 541 spaced from each other in the thickness direction. If the filter 543 in a wet condition continues to be kept in contact with the moisture detecting sensors 541, the moisture detecting sensors 541 will be maintained in the turned-on state and may malfunction to detect urination even though no urination occurs. To prevent such malfunction, the filter 543 and the moisture detecting sensors 541 are kept spaced from each other. For the purpose of preventing such malfunction, use of the spacer is particularly effective since the spacer can effectively prevent these filter 543 and moisture detecting sensors 541 from being continuously kept in contact with each other.

A dispersant sheet 549 and an air permeable-resistant sheet 550 are stacked on the side of the cushion sheet 544 facing the wearer's garment and the urine receptacle 505 is formed on the side of the air permeable-resistant sheet facing the wearer's garment. The air permeable-resistant sheet 550 and the urine receptacle 505 are joined together, for example, by adhesives.

The dispersant sheet 549 serves to disperse the discharged urine as quickly as possible and thereby to change the air permeable-resistant sheet 550 over a wide range thereof into a dry condition. As a material for the air permeable-resistant sheet 549, for example, a nonwoven fabric containing hydrophilic fibers such as rayon fibers may be used.

The air permeable-resistant sheet 550 is high in its liquid-permeability but low in its air-permeability and, as a material for this sheet 550, for example, a nonwoven fabric modified to become hydrophilic may be used.

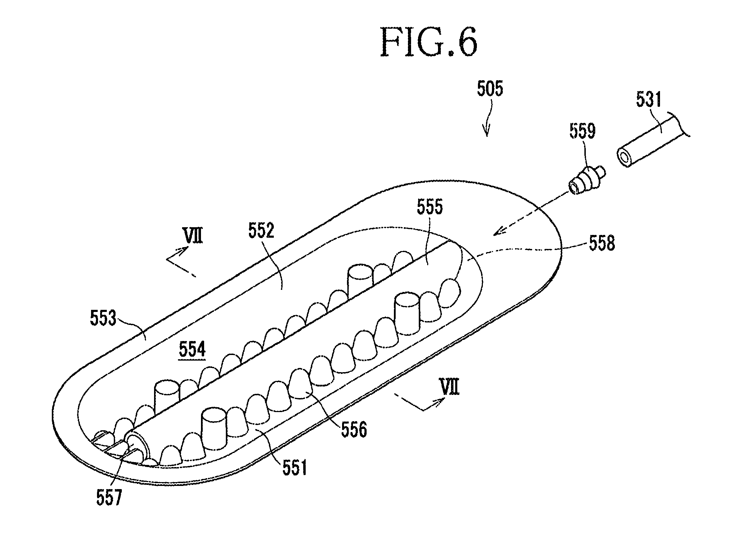

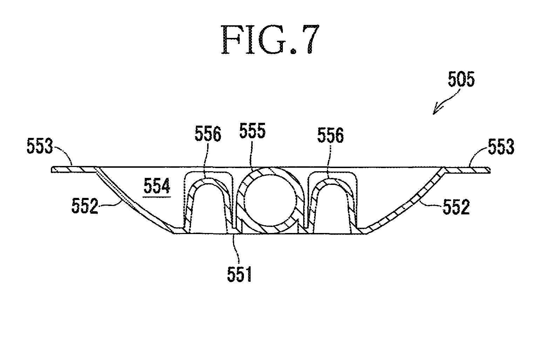

The urine receptacle 505 is flexible and may be formed, for example, of a liquid-impervious soft elastic material such as a soft polyethylene or silicon rubber. Fig. 6 is a perspective view of the urine receptacle 505 and Fig. 7 is a sectional view taken along line VII-VII in Fig. 6. The urine receptacle 505 comprises a bottom 551, a peripheral wall 552 rising from the bottom 551 toward the side of the wearer's body, and a flange 553 extending outward from a top peripheral edge of the peripheral wall 552. The flange 553 is put in contact with the air permeable-resistant sheet 550 to form a space 554 within the urine receptacle 505. The urine receptacle 505 is formed with a suction unit 555 functioning to collect the urine flowing thereinto and to evacuate this out of the urine receptacle 505. The suction unit 555 has a cylindrical shape and extends along a generally transverse middle zone of the receptacle 505 in the longitudinal direction. On both sides of the suction unit 555, a plurality of protuberances 556 extend upward from the bottom 551 toward the wearer's body. These protuberances 556 serve to prevent the air permeable-resistant sheet 550 which should be held in contact with the flange 553 from sagging down into the space 554.

Referring to Fig.5, the suction unit 555 has one end 557 opening into the urine receptacle 505 and the other end 558 opening to the outside of the urine receptacle 505. The suction tube 531 is attached to the other end 558 of the suction unit 555 with a joint 559 and the suction tube 531 is connected to the suction pump 500P.

The suction tube 531 and the suction pump 500P both connected to the other end 558 of the suction unit 555 define a moisture suction device in the present invention. Upon actuation of the suction pump 500P, the urine collected in the urine receptacle 505 is sucked through the suction tube 531. The suction pump 500P is electrically connected to the control unit 500C so that the control unit 500C detects occurrence of urination on the basis of signals transmitted from the moisture detecting sensors 541 and thereupon transmits signals to the suction pump 500P. In response to the signals from the control unit 500C, the suction pump 500P is actuated. In this way, the suction pump 500P is actuated only when the urination occurs.

Referring to Figs. 3 and 4, the moisture absorbent structure 506 is formed on the side of the urine receptacle 505 facing the wearer's garment. The moisture absorbent structure 506 comprises a moisture-pervious sheet 562 lying on the side facing the wearer's body, a liquid-impervious sheet 563 lying on the side facing the wearer's garment and moisture absorbent material 564 lyingbetween these two sheets 562, 563. The moisture absorbent material 564 comprises a moisture-absorbent core material such as fluff pulp, superabsorbent polymer particles (SAP) or silica gels wrapped with tissue paper or the like. As a material for the moisture-pervious sheet 562, for example, a thermoplastic drawn film containing inorganic fillers may be used. As the inorganic fillers, calcium carbonate, barium sulfate or the like and, as the thermoplastic film, polyethylene films or the like may be used. As both the inorganic fillers and the thermoplastic films, those widely used in the relevant technical field may be used. Moisture permeability of the moisture-pervious sheet 562 is preferably in a range of 83.3 to 166.6 $(g/m^2/24 hours)$. If the moisture permeability is lower than 83.3 $(g/m^2/24 hours)$, a significant moisture absorbing effect will not be expressed and, if the moisture permeability is higher than 166.6 $(g/m^2/24 hours)$, urine will directly infiltrate into the moisture-absorbent material. In this case, there is a possibility that the urine infiltrating into the moisture-absorbent material may cause a temperature within the wearing article to rise.

Moisture permeability was measured in accordance with JIS-Z-0208. Specifically, circular pieces each having a diameter of 0.03m were cut out from the moisture-pervious sheet as test pieces defining the boundary plane for moisture permeation. A thermo-hygrostat was set to a temperature of 40°C and a humidity of 60%. Total mass (g) of moisture vapor had passed through this boundary plane during 24 hours was converted to the mass per $1m^2$ of the tested sheet to obtain the moisture permeability.

The moisture permeability was calculated by an equation as follows:

$$\text{Permeability (Pa)} = \text{mass (g) before measurement} - \text{mass (g)}$$

$$24 \text{ hours after starting of measurement}/\pi \times 0.03 \times 0.03/24 \text{ hours.}$$

The moisture-absorbent structure 506 has a length dimension in the transverse direction X larger than that of the moisture detecting structure 504 lying on the side of the moisture-absorbent structure 506 facing the wearer's body. In other words, the moisture-absorbent structure 506 extends in the transverse direction X beyond the both ends of the moisture detecting structure 504. In the sections extending beyond the both ends of the moisture detecting structure 504, a pair of barrier cuffs 507, 507 against body waste is formed on the side of the moisture-pervious sheet 562 facing the wearer's body constituting the moisture-absorbent structure 506.

The barrier cuffs 507, 507 are attached to side edges 567, 567 of the moisture-pervious sheet, respectively, and include proximal edges 571, 571 joined to the side edges 567, 567 of the moisture-pervious sheet 562 and free edges 572, 572 lying inside of the proximal edges 571, 571 in the transverse direction X and not joined to the moisture-pervious sheet 562. These free edges 572, 572 lie on the side of the topsheet 632 facing the wearer's body and provided with cuff elastic elements 573, 573 extending in the longitudinal direction Y and attached thereto under tension and in a contractible manner. Upon contraction of the cuff elastic elements 573, 573, the free edges 572, 572 are spaced upward so as to stand on the topsheet 532 and thereby prevent urine from leaking outward in the transverse direction X. While the liquid-impervious barrier cuffs 507, 507 are effective to prevent leakage of urine, it is possible to use, for example, moisture-pervious fibrous nonwoven fabric from the viewpoint of permeability for moisture vapor.

Now the manner in which the aforementioned arrangement operates in response to occurrence of urination will be described. Upon occurrence of urination, the urine discharged infiltrates into the topsheet 532. The barrier cuffs 507, 507 formed along the side edges 533, 533 of the topsheet prevent the urine from leaking outward in the transverse direction X.
The urine having infiltrated into the topsheet 532 quickly come in contact with the moisture detecting sensors 541 through the spacer 542 and the filter 543. When urine comes in contact with the paired moisture detecting sensors 541 so as to connect these sensors with each other, these moisture detecting sensors 541 are turned on and transmit signals to the control unit 500C. In response to this signal, the control unit 500C actuates the suction pump 500P. Upon actuation of the suction pump 500P, the space 554 of the urine receptacle 505 is subjected to a suction effect via a suction unit 555.

The urine having reached the moisture detecting sensor 541 infiltrates into the dispersant sheet 549 through the cushion sheet 544. The urine having dispersant sheet 549 over a wide range simultaneously infiltrates into the air permeable-resistant sheet 550 which covers the opening of the urine receptacle 505 to define the space 554 therein. When the space 554 is subjected to the suction effect by the suction pump 500P and the opening is covered with the air permeable-resistant sheet 550 wetted with urine, a negative pressure is generated within the space 554. As a result, the urine having infiltrated in the air permeable-resistant sheet 550 is sucked into the space 554 and the urine sucked into the space 554 is sucked through the suction tube 555 from the urine receptacle 505. In this way, immediately after occurrence of urination, the discharged urine can be quickly evacuated out of the wearing article.

After the discharged urine has sucked and evacuated, substantially entire quantity of urine is evacuated from the wearing article but there is a possibility that a small quantity of urine might be left, for example, in the urine receiver 503. Particularly because the urine receptacle 505 is liquid-impervious, the possibility that a small quantity of urine might be left on its side facing the wearer's body is correspondingly high. The urine left not sucked will be vaporized by the wearer's body heat and will pervade the interior of the wearing article. However, the moisture-absorbent structure 506 formed on the side of the urine receiver 503 facing the wearer's garment is effective to absorb such moisture vapor. Specifically, moisture vapor passes through the moisture-pervious sheet 562 of the moisture-absorbent structure 506 and is absorbed by the moisture-absorbent material 564. The liquid-impervious sheet 563 lies on the side of the moisture-absorbent material 564 and therefore moisture vapor is reliably absorbed by the moisture-absorbent material 564 without leaking out. Even if moisture vapor is generated within the wearing article 501, such moisture vapor can be reliably absorbed by the moisture-absorbent material 564. In consequence, there is no possibility that a temperature within the pants might rise and the wearer might suffer from any skin troubles or a discomfortable feeling due to the undesirable humidity.

The moisture absorbent structure 506 is dimensioned in the transverse direction X to be larger than that of the moisture detecting structure 504. With such dimensioning, moisture vapor can be absorbed by the section extending outward beyond the periphery of the urine receiver 503 and therefore the absorbing function should not be disturbed by various sheets of the urine receiver 503.

Even if moisture vapor within the wearing article 501 increases due not only urine but also to the wearer's sweat of the wearer, the moisture-absorbent structure 506 can effectively absorb moisture vapor and thereby restrict increase of temperature.

While the wearer's article is provided with the moisture detecting sensor 541 serving for detection of urine according to the present embodiment, it is possible to incorporate, in addition to the moisture detecting sensor 541, a feces detecting senor. While the mesh pants are used as the chassis 502 according to the present embodiment, it is also possible to use widely used means such as disposable diapers or diaper covers as the chassis 502.

## Claims

1. A wearing article (501) having a longitudinal direction and a transverse direction, comprising a side facing the wearer's body and a side facing the wearer' garment, a chassis (502) having a front waist region (521), a rear waist region (522) and a crotch region (523) extending between said front and rear waist regions and a receiver (503) adapted to receive bodily fluids discharged onto said chassis wherein the receiver is arranged such that the bodily fluids received by said receiver may be sucked by a moisture suction device (500P) out of the wearing article, wherein said receiver comprises a receptacle (505) connected to said moisture suction device to collect the bodily fluids received by said receiver, said wearing article being **characterized in that**:

   said receiver further comprises a moisture-absorbent structure (506) lying on the side of said receptacle facing the wearer's garment; and said moisture-absorbent structure comprises a moisture-pervious sheet (562) lying on the side of said moisture absorbent structure facing said wearer's body, a moisture-impervious sheet (563) lying on the side of saidmoisture absorbent structure facing the wearer's garment and a moisture-absorbent material (564) sandwiched between said moisture-pervious sheet and said liquid-impervious sheet.

2. The wearing article according to claim 1, wherein, said receiver additionally includes a moisture detecting structure (504) comprising a moisture detecting sensor (541), a spacer (542) lying on the side of said moisture detecting sensor facing the wearer's body, a dispersant sheet (549) lying on the side of said moisture detecting sensor facing the wearer's garment and an air permeable-resistant sheet (550) lying on the side of said dispersant sheet facing the wearer's garment.

3. The wearing article according to claim 1 or 2, wherein said moisture-absorbent structure has a length in the transverse direction dimensioned to be longer than that of said moisture detecting structure to extend outward in the transverse direction beyond the side edges of said moisture detecting structure.

4. The wearing article according to any one of claims 1 through 3, wherein said moisture detecting structure extends in the longitudinal direction from said front waist region to said rear waist region.

5. The wearing article according to claims 1 through 4, wherein said receptacle is liquid-impervious and flexible and positioned in said rear waist region in use of said receptacle.

## Patentansprüche

1. Kleidungsstück (501), das eine Längsrichtung und eine Querrichtung hat, und eine Seite umfasst, die dem Körper des Trägers zugewandt ist, und eine Seite, die dem Kleidungsstück des Trägers zugewandt ist, wobei ein Rahmen (502) mit einem vorderen Taillenbereich (521), einem hinteren Taillenbereich (522) und einem Schrittbereich (523) hat, die sich zwischen dem vorderen und hinteren Taillenbereich erstrecken, und einen Empfänger (503), der so bearbeitet wurde, dass er Körperflüssigkeiten empfängt, die auf den Rahmen ausgeschieden werden, wobei der Empfänger so angeordnet ist, dass die Körperflüssigkeiten, die vom Empfänger aufgenommen werden, von einem Wasseraufnahmegerät (500P) vom Kleidungsstück herausgesaugt werden können, wobei der Empfänger einen Behälter (505) umfasst, der am Wasseraufnahmegerät angeschlossen ist, um die Körperflüssigkeiten zu sammeln, die vom Empfänger erhalten wurden, und das Kleidungsstück **dadurch gekennzeichnet ist, dass** der Empfänger ferner eine feuchtigkeitsabsorberende Struktur (506) umfasst, die auf der Seite des Behälters liegt und dem Kleidungsstück des Trägers zugewandt ist, und die feuchtigkeitsabsorbierende Struktur eine wasserdurchlässige Schicht (562) umfasst, die auf der Seite der feuchtigkeitsabsorbierenden Struktur liegt und dem Körper des Trägers zugewandt ist, eine wasserundurchlässige Schicht (563), die auf der Seite der feuchtigkeitsabsorbierenden Struktur liegt und dem Kleidungsstück des Trägers zugewandt ist, und ein feuchtigkeitsabsorbierendes Material (564), das zwischen der wasserdurchlässigen Schicht und der wasserundurchlässigen Schicht eingelegt ist.

2. Kleidungsstück nach Anspruch 1, wobei der Empfänger zusätzlich eine die Flüssigkeit erkennende Struktur (504) beinhaltet, die einen die Feuchtigkeit erkennenden Sensor (541), ein Abstandsstück (542), das auf der Seite des die Feuchtigkeit erkennenden Sensors liegt, der dem Körper des Trägers zugewandt ist, eine zerstreuende Schicht (549), die auf der Seite des die Feuchtigkeit erkennenden Sensors liegt und dem Kleidungsstück des Trägers zugewandt ist, und eine luftdurchlässige Schicht (550) umfasst, die dem Kleidungsstück des Trägers zugewandt ist.

3. Kleidungsstück nach Anspruch 1 oder 2, wobei die feuchtigkeitsabsorbierende Struktur eine Länge in Querrichtung hat, die so dimensioniert ist, dass sie länger als die der Feuchtigkeit erkennenden Struktur ist, und sich nach außen in Querrichtung über die Seitenränder der die Feuchtigkeit erkennenden Struktur erstreckt.

4. Kleidungsstück nach einem der Ansprüche 1 bis 3, wobei die eine Feuchtigkeit erkennende Struktur sich in Längsrichtung vom vorderen Taillenbreich bis zum hinteren Taillenbereich erstreckt.

5. Kleidungsstück nach Anspruch 1 bis 4, wobei der Behälter wasserundurchlässig und flexibel ist, und im hinteren Taillenbereich bei Verwendung des Behälters ist.

**Revendications**

1. Article vestimentaire (501) ayant une direction allant dans le sens longitudinal et une direction allant dans le sens transversal, comportant un côté orienté vers le corps de l'utilisateur et un côté orienté vers le vêtement de l'utilisateur, une armature (502) ayant une région avant au niveau de la taille (521), une région arrière au niveau de la taille (522) et une région au niveau de l'entrejambe (523) s'étendant entre lesdites régions avant et arrière au niveau de la taille et un récepteur (503) adapté à des fins de réception de fluides organiques déchargés sur ladite armature dans lequel le récepteur est agencé de telle sorte que les fluides organiques reçus par ledit récepteur peuvent être aspirés par un dispositif d'aspiration d'humidité (500P) en provenance de l'article vestimentaire, dans lequel ledit récepteur comporte un contenant (505) raccordé audit dispositif d'aspiration d'humidité à des fins de collecte desdits fluides organiques reçus par ledit récepteur, ledit article vestimentaire étant **caractérisé en ce que** :

   ledit récepteur comporte par ailleurs une structure d'absorption d'humidité (506) reposant sur le côté dudit contenant orienté vers le vêtement de l'utilisateur ; et
   ladite structure d'absorption d'humidité comporte une feuille perméable à l'humidité (562) reposant sur le côté de ladite structure d'absorption d'humidité orienté vers le corps de l'utilisateur, une feuille imperméable à l'humidité (563) reposant sur le côté de ladite structure d'absorption d'humidité orienté vers le vêtement de l'utilisateur et un matériau d'absorption d'humidité (564) pris en sandwich entre ladite feuille perméable à l'humidité et ladite feuille imperméable aux fluides.

2. Article vestimentaire selon la revendication 1, dans lequel ledit récepteur comprend aussi une structure de détection d'humidité (504) comportant un capteur de détection d'humidité (541), un élément d'espacement (542) reposant sur le côté dudit capteur de détection d'humidité orienté vers le corps de l'utilisateur, une feuille de dispersion (549) reposant sur le côté dudit capteur de détection d'humidité orienté vers le vêtement de l'utilisateur et une feuille résistance perméable à l'air (550) reposant sur le côté de ladite feuille de dispersion orienté vers le vêtement de l'utilisateur.

3. Article vestimentaire selon la revendication 1 ou la revendication 2, dans lequel ladite structure d'absorption d'humidité a une longueur dans la direction allant dans le sens transversal dimensionnée pour être plus longue que celle de ladite structure de détection d'humidité à des fins d'extension vers l'extérieur dans la direction allant dans le sens transversal au-delà des bords latéraux de ladite structure de détection d'humidité.

4. Article vestimentaire selon l'une quelconque des revendications 1 à 3, dans lequel ladite structure de détection d'humidité s'étend dans la direction allant dans le sens longitudinal depuis ladite région avant au niveau de la taille jusqu'à ladite région arrière au niveau de la taille.

**5.** Article vestimentaire selon l'une quelconque des revendications 1 à 4, dans lequel ledit contenant est imperméable aux liquides et flexible et est positionné dans ladite région arrière au niveau de la taille lors de l'utilisation dudit contenant.

# FIG.1

EP 2 380 532 B1

# FIG.2

# FIG.3

# FIG.4

# FIG.5

# FIG.6

EP 2 380 532 B1

# FIG.7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8510924 A
- JP 2006026108 A

- US 20070038194 A